# EUROPEAN PATENT SPECIFICATION

(11) **EP 4 712 833 B1**
(45) Date of publication and mention of the grant of the patent: **21.10.2026**
(21) Application number: 24728710.5
(22) Date of filing: 25.04.2024
(51) Int. Cl.: A61B 3/113, G06F 3/01, G02C 7/04, G02B 27/00, G02C 7/02

(54) **USER'S EYE TRACKING METHOD AND CONTACT LENS**
VERFAHREN ZUR VERFOLGUNG DES AUGES EINES BENUTZERS UND KONTAKTLINSE
PROCÉDÉ DE SUIVI DES YEUX D'UN UTILISATEUR ET LENTILLE DE CONTACT

(30) Priority: 14.07.2023 RU 2023118660
(43) Date of publication of application: 25.03.2026
(73) Proprietor: XPANCEO RESEARCH ON NATURAL SCIENCE L.L.C. S.O.C., Dubai (AE)
(72) Inventor: ARSENIN, Aleksey Vladimirovich, Moscow, 111672 (RU); VOLKOV, Valentin Sergeevich, Dolgoprudnyy, 141700 (RU); SOLOVEI, Valentin Romanovich, Moscow, 127254 (RU); SYUY, Alexander Vyacheslavovich, Moscow, 127204 (RU); FRADKIN, Ilia Markovich, Moscow, 127562 (RU)
(74) Representative: COPA Copenhagen Patents
(86) International application number: PCT/RU2024/050090
(87) International publication number: WO 2025/018917

(56) References cited:
- EP-B1- 1 066 717
- CA-C- 1 317 490
- US-B1- 11 448 899
- US-B2- 11 393 435

## Description

### Technical Field

The group of inventions relates to the field of wearable optics, and, in particular, to the user's eye tracking methods, and to the contact lenses enabling implementation of said method, and can be used in the augmented, virtual, mixed, or extended reality (AR/VR/MR/XR) forming systems.

### Background Art

The prior art discloses a method of measuring a camera inclination angle to a reference plane provided with an image of a periodic lattice, consisting in carrying out an analysis of moire fringes formed when a second periodic lattice is laid over said image, following which the relative inclination angle of the two lattices and the camera viewing angle are calculated (patent CN107808399B, cl. G06T7/80, published 26.10.2021). The main disadvantage of the prior art method is that it can only be used to determine the camera position relative to some object, but not the position of the object of interest relative to the camera.

The closest, in terms of technical substance, to the claimed invention as related to the method is the user's eye tracking method according to which a plurality of cameras are installed in an eyeglass frame to enable monitoring of the user's eyes, a contact lens is placed on the user's eye, and the user's eyes position is determined from the position of the eye with the contact lens relative to the user's head (patent US11393435B2, cl. G02B27/00, published 19.07.2022). The main disadvantages of the prior art method are potentially low accuracy in determining the position of the eyes and complexity to implement such a method: it is necessary to use glasses with a plurality of cameras built therein, and the contact lens should be equipped with a camera for tracking the user's gaze direction.

The closest, in terms of technical substance, to the claimed invention as related to the device is a cosmetic contact lens containing a central region that in operative position is located opposite the user's pupil and a peripheral region located outside the user's field of view, at least in part made of a transparent material and provided with a multilayer structure creating a "sparkling" moire pattern by superimposing periodic patterns of different layers (patent CA1317490C, cl. G02C7/04, published 11.05.1993). The main disadvantage of the prior art device is its limited functionality defining its use for cosmetic purposes only.

The technical problem that is solved by the disclosed inventions is the necessity to eliminate the above disadvantages and to create a comparatively simple and cheap to manufacture system providing high accuracy in determining the user's eyes position relative to the camera. Furthermore, it is reasonable to apply a high-accuracy method of determining a normal to the surface of different parts of the contact lens to dynamically determine its shape and, accordingly, indirectly determine intraocular pressure.

### Summary of Invention

The technical effect consists in enhancing the accuracy when measuring the position of the contact lens and, hence, of the user's eyes.

The set problem has been solved and the technical effect has been achieved, as related to the method, by that according to the disclosed user's eyes tracking method a camera is installed that is configured to monitor the user's eyes, at least on the user's one eye a contact lens is placed that is equipped with an optical structure forming a stereoscopic image, and the user's eyes position is determined from the linear dimension of this image recorded by the camera and from the angular position of this image relative to the optical axis of said camera. Said optical structure is preferably implemented as a hologram or, alternatively, as an autostereogram with a decoding element that are separated by a layer of transparent material. To determine the intraocular pressure, said optical structure is implemented in the form of at least two segments located along the contact lens perimeter, the angular position of the formed stereoscopic image is determined based on each of said segments, and the obtained data is used to determine the contact lens bending radius.

The set problem has been solved and the technical effect has been achieved, as related to the device according to the first embodiment, by that in the contact lens containing a central region that in operative position is located opposite the user's pupil and a peripheral region located outside the user's field of view, at least in part made of a transparent material, and equipped with an optical structure, said optical structure is implemented as an autostereogram and a decoding slit raster that is formed on the outer surface of the contact lens peripheral region and configured to form stereoscopic image encoded in said autostereogram, wherein the autostereogram is formed by reflecting said slit raster in a mirror coating applied on the inner surface of the contact lens peripheral region, or said autostereogram is formed by a shadow cast by said slit raster on a diffusely reflecting coating applied on the inner surface of the contact lens peripheral region.

The set problem has been solved and the technical effect has been achieved, as related to the device according to the second embodiment, by that in the contact lens containing a central region that in operative position is located opposite the user's pupil and a peripheral region located outside the user's field of view, at least in part made of a transparent material, and equipped with an optical structure, said optical structure is implemented as an autostereogram and a decoding raster configured to form stereoscopic image encoded in said autostereogram, which are separated by a layer of transparent material, wherein said autostereogram and/or decoding raster are formed as a real image comprised of replicated elements with the period *p* and at least of two different characteristic linear dimensions, at least one of which is *d ₁ <1*/*2-p.* Said replicated elements are preferably configured to form stereoscopic image adapted to be used as a goniometric scale. Said peripheral region of the contact lens preferably extends beyond the user's eye iris.

The set problem has been solved and the technical effect has been achieved, as related to the device according to the third embodiment, by that in the contact lens containing a central region that in operative position is located opposite the user's pupil and a peripheral region located outside the user's field of view, at least in part made of a transparent material, and equipped with an optical structure, said optical structure is implemented as a hologram recorded for a given light wavelength and configured to form a stereoscopic image when the contact lens is illuminated by a beam with said light wavelength.

### Brief Description of Drawings

**Fig.1**

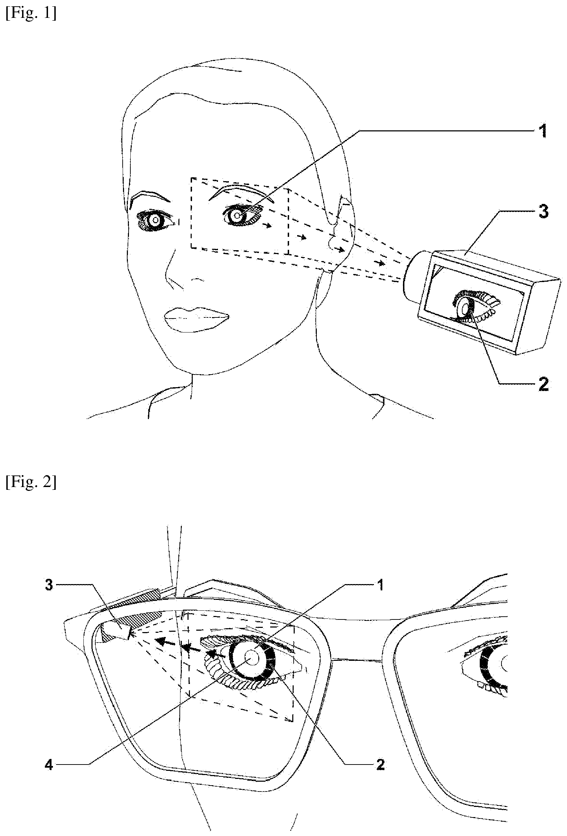

   [Fig.1] shows a disclosed method realization scheme in general;
**Fig.2**
   [Fig.2] shows an embodiment of the method using a camera installed on an eyeglass frame;
**Fig.3**

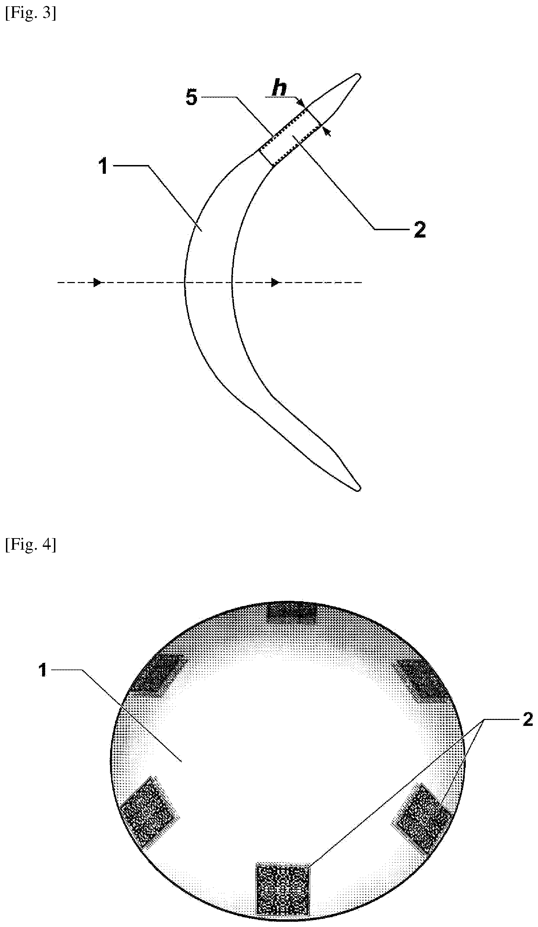

   [Fig.3] shows a contact lens enabling realization of said method using an optical structure comprised of a decoding element in the form of a slit raster and an autostereogram in the form of a real image (in axial section);
**Fig.4**
   [Fig.4] shows a contact lens enabling realization of said method using an optical structure comprised of a decoding element in the form of a coded aperture (general view);
**Fig.5**

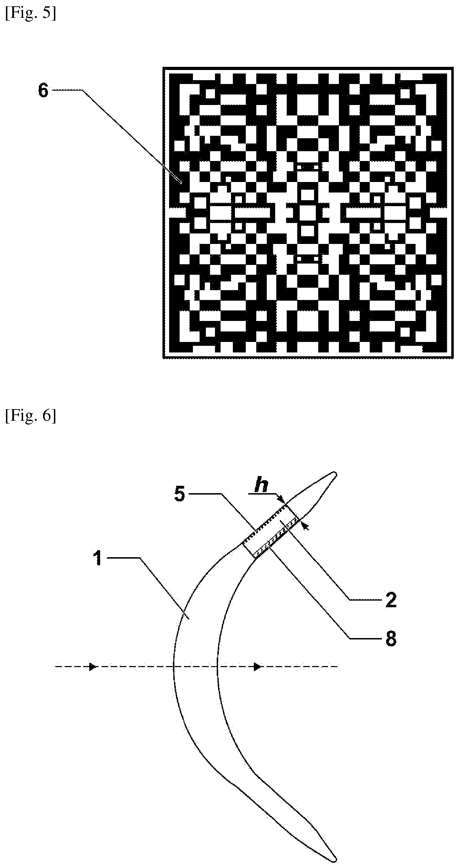

   [Fig.5] shows an embodiment of such a coded aperture;
**Fig.6**
   [Fig.6] shows the contact lens according to the first embodiment with an autostereogram in the form of a reflection in a mirror coating or a shadow on a diffusely reflecting coating (in axial section);
**Fig.7**

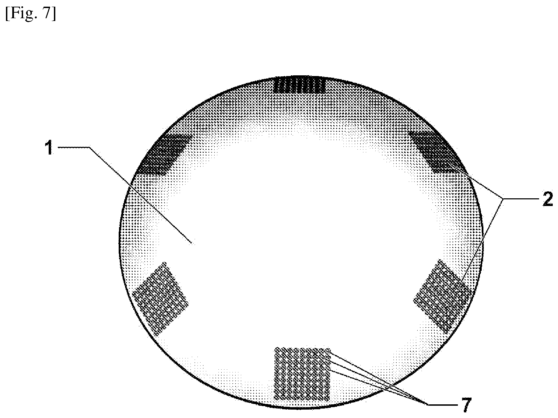

   [Fig.7] shows the disclosed contact lens according to the second embodiment with an optical structure comprised of an autostereogram in the form of a real image and a decoding raster in the form of an array of microlenses (general view);
**Fig.8**

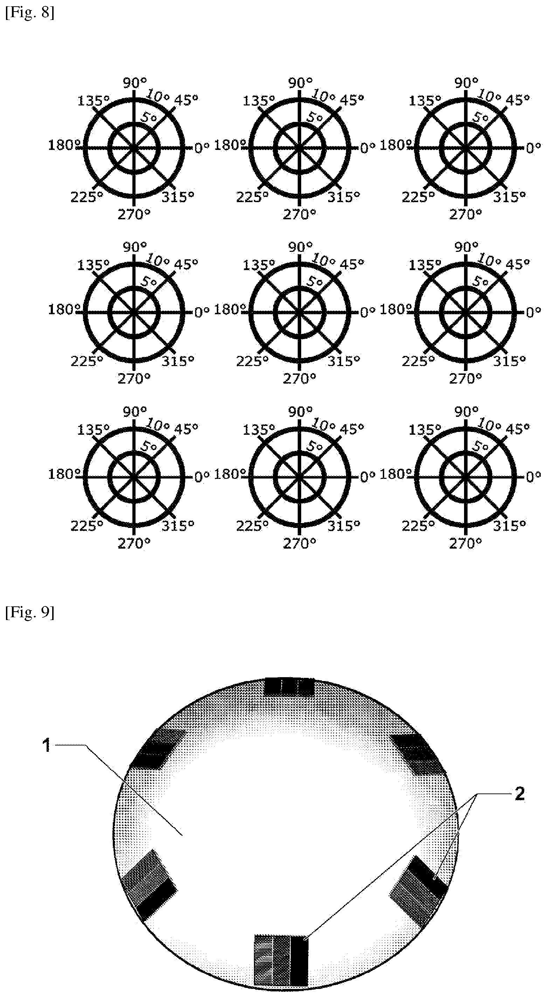

   [Fig.8] shows an image portion for the autostereogram of [Fig.7] in the form of a goniometric scale;
**Fig.9**
   [Fig.9] shows the disclosed contact lens according to the second embodiment with an optical structure comprised of an autostereogram and a decoding raster in the form of real images separated by a layer of transparent material;
**Fig.10**

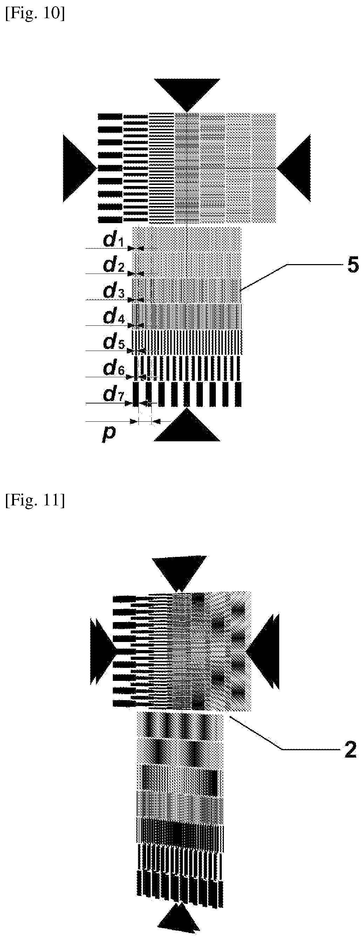

   [Fig.10] is an example of an image for the optical structure of [Fig.7] in the form of a pair of orthogonally oriented lattices comprised of replicated elements having seven different characteristic linear dimensions;
**Fig.11**
   [Fig.11] shows a camera recorded image formed by the optical structure of [Fig. 10] at the observation angle of 45 degrees and distance of 3 cm;
**Fig.12**

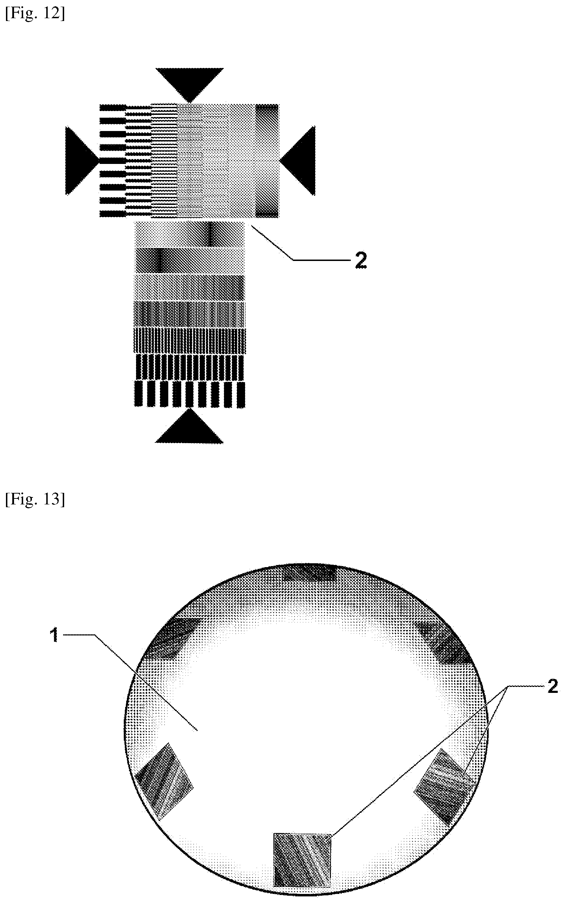

   [Fig.12] shows a camera recorded image formed by the optical structure of [Fig.10] at the observation angle of 15 degrees and the distance of 10 cm;
**Fig.13**
   [Fig.13] shows the disclosed contact lens according to the third embodiment with an optical structure in the form of a hologram (general view);
**Fig.14** **-** **Fig.16**

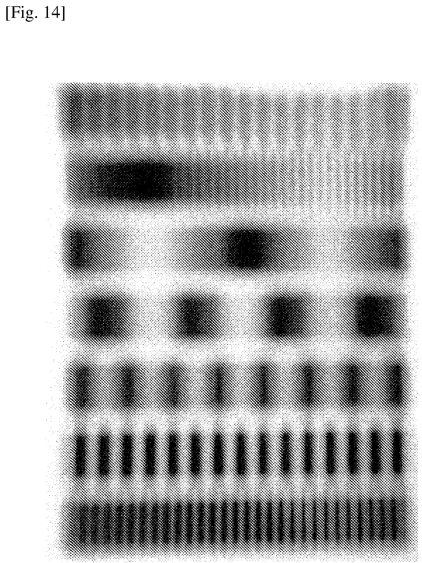

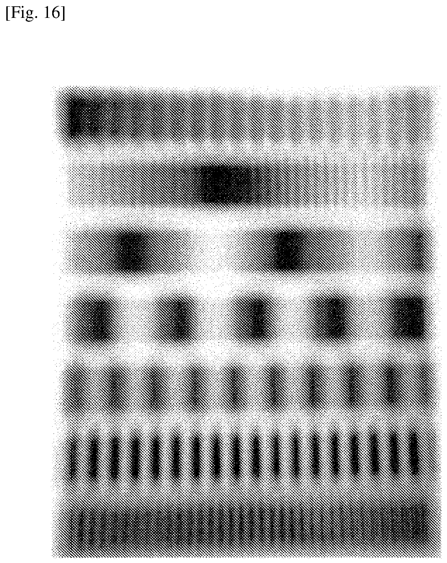

   [Fig.14] - [Fig.16] show the photographs of a series of moire fringes from an optical structure in the form of the two real images of diffraction lattices separated by a layer of transparent material that are obtained using a mobile phone camera at different shooting angles and from different distances;
**Fig.17**

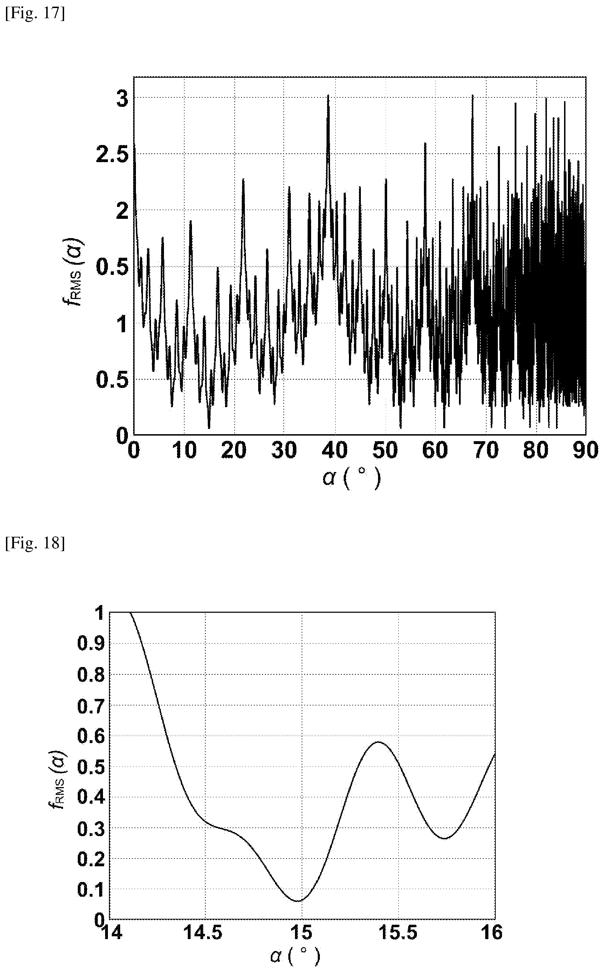

   [Fig.17] shows a graph of squared difference of intensity i *ₙ*^{photo} of the moire fringes recorded using the camera, and the respective theoretically estimated intensity *i ₙ*(*α*) as a function of the optical structure inclination angle towards the camera optical axis - function *f*_{RMS}(*α*), within the angular range from 0 to 90 degrees;
**Fig.18**
   [Fig.18] is the same as [Fig.17] within the angular range from 14 to 16 degrees.

### Description of Embodiments

The disclosed eye tracking method consists essentially in using a pair (to track both eyes) or one (to track one eye) contact lens 1 having the multilayer optical structure 2 that forms a stereoscopic image for the camera 3 [Fig.1].

The camera 3 can be disposed on the eyeglass frame [Fig.2] (on the edge of the glasses temple, glasses frame rim, hinge, flex, end piece, etc.), in a mobile phone, fixed on the body of a laptop, video display, office or home furniture, or in any other accessible place making it possible to reliably monitor the user's eyes. If the camera 3 is mounted rotatably, it is additionally equipped with a device determining the position of its own optical axis and the degree of rotation about this axis.

To avoid limiting the field of view, the contact lens 1 contains the central region that in operative position is located opposite the user's pupil 4 and a peripheral region located outside the user's field of view. The peripheral region is partially or entirely made of a transparent material and it is the region that is equipped with the optical structure 2. To ensure better visibility (against the white background of the white of the eye), the peripheral region is made extended beyond the user's iris.

Once such a contact lens 1 is put on the user's eye, the eye position (gaze direction) is determined (reconstructed) from the contact lens 1 position and orientation in space. The contact lens 1 surface position, in its turn, is determined from the position of one complex or several simple optical structures 2. The optical structure 2 preferably consists of several segments disposed along the perimeter of the contact lens 1: in this way the normals to the surface of the lens 1 can be determined in its different parts. This makes it possible to correctly find the eyes position even if the contact lens 1 has rotated to a certain angle, and to enhance the measuring accuracy. By providing sufficient accuracy it is possible to determine a change in the contact lens 1 bending radius (by determining the angular position of the formed stereoscopic image for two or more segments).

Said optical structure 2 can be implemented as a holographic optical element (hologram), computer hologram (a metasurface designed using special computational methods and produced by means of lithography, possessing the properties similar to a real hologram, i.e. forming a certain stereoscopic image under external illumination), multilayer structure, etc.

In the simplest case, the optical structure is implemented as an autostereogram (autostereography is a system of techniques and methods that makes it possible to use a flat image to recreate an illusion of a three-dimensional image) and a decoding element (for example, a slit raster 5 [Fig.3] or a coded aperture 6 [Fig.4]).

When the decoding element is located at a certain distance (equal to the thickness of transparent material layer of the contact lens 1) from the autostereogram, the respective different parts of the autostereogram can be seen through the decoding element while observing the optical structure 2 under different angles. Thus, the structure of the object acting as an autostereogram defines the angular dependence of the light intensity (and, potentially, its spectrum) and can be used to design a required stereoscopic image. If the stereoscopic image is located at infinity, then every part of the stereoscopic image should be visible strictly at its respective angle regardless of which part of the autostereogram is observed. In this case, the autostereogram designing is quite trivial - it is just necessary to use a periodic decoding element and replicate the infinity projected image with the period of this decoding element at a corresponding scale, following which to focus the camera 3 on infinity. Then, in any part of the periodic decoding element, only the image part that corresponds to the observation angle will be visible. Hence, it becomes apparent that the smaller is this part (for example, the raster 5 slit width) with respect to the period, the higher is the angular resolution of the stereoscopic image, but the lower are its brightness and contrast.

As an alternative explanation, each slit of the raster 5 can be regarded as a camera obscura that projects a small image from the autostereogram to infinity. Since in one separate slit of the raster 5 we can only see a small part of the stereoscopic image of interest, to observe all of the image parts, the same structure should be replicated many times, which results in forming a lattice.

Based on the two explanations, it becomes apparent that it is not necessary at all to make the autostereogram and the decoding element strictly periodic structures as in [Fig.3] - it is only important that their respective parts are aligned with each other (for example, as in the case with the coded aperture 6 [Fig.4], [Fig.5]).

The decoding raster can be represented by a lattice of slits (the slit raster 5 - [Fig.3], [Fig.6], [Fig.9]) or of pinholes, cylindrical or spherical microlenses 7 (lenticular raster) [Fig.7], etc. The autostereogram can be implemented in the form of a periodically, quasiperiodically, aperiodically replicated real image, and also in the form of a mirror reflection or a shadow of a decoding raster (for example, the slit raster 5) on the coating 8 [Fig.6] that is either mirroring or diffusely reflecting accordingly, etc.

In the simplest case, the optical structure 2 consists of two or more strictly periodic, gradient (apodized) lattices, aperiodic or quasi-crystalline structures, separated by a layer of transparent material [Fig.3]. In the case of two lattices, one of them (formed using the contact lens 1 inner surface) acts as an autostereogram, and the other (formed on the contact lens 1 external surface) acts as a decoding element (for example, the slit raster 5). Alternatively, the optical structure 2 can be implemented as a separately produced multilayer structure that is completely integrated inside the contact lens 1 (not shown in the drawings), which does not limit the subject matter of the embodiments disclosed below.

Since the stereoscopic image is formed at some (potentially, infinite) distance from the optical structure 2, the optical structure 2 can be regarded as an aperture ("window") limiting the visible area of the stereoscopic image. Knowing which part of the stereoscopic image exactly is recorded by the camera 3, it is possible to estimate the relative positions of the camera 3 and the optical structure 2 and, consequently, to determine the distance between them and the angle between the normal to the surface of the optical structure 2 and the optical axis of the camera 3.

In the contact lens 1 according to the first embodiment, the autostereogram is formed by said slit raster 6 being reflected in the coating 8 [Fig.6] that is implemented as mirroring. Alternatively, the autostereogram can be formed by a shadow cast by said slit raster 6 on the coating 8 that is implemented as diffusely reflecting (diffusive surface). The coating 8 can be applied onto the inner surface of the contact lens 1 peripheral region or disposed in a cross section plane of the contact lens. The disclosed embodiment has a number of advantages described below.

In the case of reflection, the formed mirror image of the decoding slit raster 5 is located two times further from it than the mirror plane of the coating 8 (which, accordingly, doubles sensitivity to angle variation for the given dimensions of the optical structure 2). As the thickness of the contact lens 1 in practice is limited by several hundreds of micrometers, the effective increase of its thickness by two times is an apparent advantage that potentially makes it possible to increase the user's eye position and gaze direction determination accuracy by two times as well. The second apparent advantage is avoiding the necessity to align the autostereogram image and the decoding slit raster 5 - provided that the parallel alignment of all the planes is maintained, the mirror image in the surface 8 is automatically aligned with the raster 5 being mirrored.

Using a shadow as an autostereogram partly solves the alignment problem as well. In this case, the shadow lines on the surface 8 are likewise parallel to the lines of the source slit raster 5, but displaced to a certain distance depending on the structure exposure angle. Also, using a shadow makes it possible to increase the image contrast. In particular, this can be achieved by using a bright external source of light with a small angular size or a monochromatic source with a respective wavelength filter for the camera 3.

In the contact lens 1 according to the second embodiment, the optical structure 2 is implemented as an autostereogram and a raster-type decoding element (not necessarily the slit raster 5 - it can be, for example, a lenticular raster or an array of microlenses [Fig.7]) that are separated by a layer of transparent material. At least one of these elements (for example, the autostereogram) should be formed as a real image, which can be obtained, for example, by applying a diffusely scattering or another material that is contrasting to the background. To increase the stereoscopic image angular resolution and the eye position measuring accuracy, the autostereogram should be formed by replicated elements with the period p and at least two different characteristic linear dimensions *d ₁* ... *d _{N}* [Fig.10]. At least one of said linear dimensions should satisfy the inequation *d ₁ <1*/*2-p.* This is due to the fact that the elements smaller than half a period naturally result in formation of fine details of the stereoscopic image, which in their turn can be used to determine mutual orientation of the optical structure 2 and the camera 3 with greater accuracy. Interaction between the decoding raster and the autostereogram results in formation of a stereoscopic image encoded in said autostereogram. The angle of its observation from different sides can be easily determined from the image on the camera 3. For the autostereogram and the decoding slit raster 5 in the form of a pair of orthogonally oriented lattices formed by replicated elements having seven different characteristic linear dimensions (the common period *p* is the period of the largest elements), the stereoscopic image represents a series of moire fringes. When the camera 3 observes the moire fringes [Fig.11], [Fig.12] under different angles and from different distances, different parts thereof will be seen. Comparing the moire fringe phase between different sublattices, it is possible to determine which part of the common stereoscopic image specifically is observed by the camera 3 and, consequently, under which angle the optical axis of the camera is inclined towards the optical structure 2. Based on the visible range of the spectral image, it is possible to reconstruct the distance from the camera 3 to the optical structure 2.

The disclosed contact lens 1 according to the second embodiment makes it possible to choose the decoding raster and the autostereogram independently of each other and, hence, to obtain almost arbitrary stereoscopic image. In particular, despite the technical complexity of implementation, the choice of certain stereoscopic images can make interpretation of the obtained data far more simple. For example, the autostereogram can be represented by a real image of a goniometric scale [Fig.8] on which the polar and azimuthal angles are denoted by marks and values. To reconstruct such an autostereogram, it is practical to use a raster represented by an array of microlenses 7 [Fig.6]. In this case, the observer or camera 3 will "see" directly under which angle the optical structure 2 is disposed towards them.

In the contact lens 1 according to the third embodiment, the optical structure 2 is implemented as a hologram [Fig.13]. Such a hologram can be most easily recorded directly on the lens containing photorefractive material that is sensitive to a given light wavelength (for example, 532 nm). The wavelength is selected depending on the assumed scheme of subsequent reconstruction in the visible spectrum (under normal illumination) and beyond its limits (for the IR or UV light), wherein the reconstruction should be performed using the respective light source.

Such an embodiment makes it possible to avoid difficulties with separate production of the autostereogram and the decoding element, as the optical structure 2 forming the required stereoscopic image is obtained at once using a single hologram recording procedure.

With all the described advantages, in practice, reconstructing orientation of the optical structure 2 based on the image observed by the camera 3 is a separate data processing task. The general idea of parameter reconstruction can be described by the following example.

### Examples

The image obtained on the camera 3 is compared with a theoretical estimation of how the optical structure 2 should appear at different observation angles. Then, based on the closest theoretical estimation, the camera 3 angle and distance from the optical structure 2 are reconstructed. Depending on the complexity of the optical structure 2, on the stereoscopic image encoded therein, as well as on relative position of the structure 2 and the camera 3, the theoretical analysis of the image can be carried out either purely analytically or through numerical simulation, using advanced image processing and analyzing techniques and subsequent postprocessing.

Below described is one of the most simple optical structures 2 represented by one lattice (comprising several sublattices with different periods, consisting of the elements with different linear dimensions) and by another similar lattice disposed at a distance H. Such a structure can indeed consist of two identical lattices [Fig.3], as well as it can alternatively be formed by one lattice and its mirror image [Fig.6]. Such a lattice can be used in practice and also makes it possible to relatively easily reconstruct orientation of the optical structure 2 with respect to the camera 3.

The optical structure 2 in which the image is obtained as a result of superimposing the lattice and its shadow has a similar principle of operation. However, when reconstructing the angle of rotation of such an optical structure 2, is it necessary to take into account the position of the light source, which somewhat complicates the data processing, and for this reason the corresponding description is not given.

Let us assume that the optical structure 2 consists of several slit rasters 5 [Fig.10] represented by the lattices with different periods, applied onto the external surface of the contact lens 1. The lattices are chosen so that the lattice under number *n*∈{ *1, ...N* } has the period *p ₙ = p*₁2^{*n*-1}. It is essential that all the lattices are aligned so that the midpoints of the central lines (slits) of each lattice are on the same line. To reconstruct orientation of the plane of the optical structure 2 in all directions, it is practical to use a pair of identical series of lattices oriented in orthogonal directions - i. e. rotated 90 degrees relative to each other.

On the contact lens 1 inner surface, the mirror coating 8 is applied. Assuming that locally on the scale of the optical structure 2 the contact lens 1 represents a plane-parallel plate with the thickness *h* [Fig.6], at the distance *H = 2h* from the source lattice we obtain the autostereogram in the form of a reflection of the decoding slit raster 5. Alternatively, the autostereogram can be produced at the distance *H =* h and directly as a real image that is equivalent to the decoding slit raster 5 [Fig.3], without using the coating 8. In this case, all the subsequent results will be the same provided that the distance between the components is substituted by the smaller one of the optical structure 2.

When observing such an optical structure 2 from the camera 3 side, we will see moire patterns resulting from superimposition of the source lattice and its reflection. This phenomenon results from the fact that different parts of the optical structure 2 are seen under different angles, but at the same time, due to the parallax effect, under different observation angles the lattice lines are superimposed on one another in different phases, and this results in periodical alternation of dark and light moire fringes.

In paraxial approximation for a pair of lattices with the same period *p ₙ*, the period of the moire fringes in the space of angles will correspond to
[Math. 1] ${p}_{n}^{a} = \frac{{p}_{n}}{H}$

Accordingly, in the discussed geometry, the period of the moire fringes is proportional to the period of lattices, and, in its turn, is proportional to the powers of two.

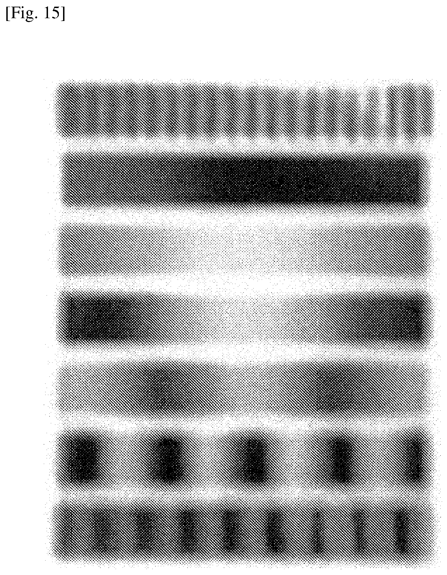

[Fig.14], [Fig.15], [Fig.16] show the photographs of a lithographically formed optical structure consisting of two identical titanium lattices separated by a glass layer, under a series of different shooting angles and from different distances. The lattices are displaced relative to each other to a random distance, due to which fact the moire pattern is shifted in the angular space to a corresponding angle. The optical structure 2 is illuminated by diffused light from the back side to increase the pattern contrast, and the camera 3 is focused on infinity. The photographs demonstrate clearly distinguishable lines of moire fringes with different periods.

During the relative movement of the camera 3, the following phenomenon is observed: on the obtained video, the moire fringes remain stationary as if they really were at infinity. At the same time, the area of the whole optical structure acts as a "window" (limited by its framework of the visible region), which makes it possible to see one or another part of the distantly located stereoscopic image of the moire fringes. From the fringe intensity ratio in each image section, it is possible to determine under which angle the respective part of the optical structure 2 is inclined towards the optical axis of the camera 3. From the number of the moire fringes that fit within the area of the optical structure 2, it is possible to estimate its angular size (aperture), and, knowing the lattice size, it is easy to reconstruct its distance to the camera 3.

Accordingly, for example, when the camera 3 is approaching the optical structure 2, we can see a greater number of fringes (the "window" aperture increases, while the angular period of the moire fringes is constant). Based on the number of fringes that fit the "window" with a known width, it is easy to reconstruct the distance to the lattice. In paraxial approximation, the distance D between the camera 3 and the optical structure 2 can be found as
[Math. 2] $D = \frac{w}{{M}_{n} {p}_{n}^{a}} = \frac{wH}{{M}_{n} {p}_{n}}$
where w is the lattice width, *H* is the distance between the lattices, *M ₙ* is the number of moire fringes for the n-th lattice, and p *ₙ* is the lattice period.

Each individual pair of lattices with a small period cannot be used as such to determine the angle in a wide range as the respective moire pattern is periodic. In this case, any, no matter how advanced data processing will give the rotation angle value with the accuracy up to one or several periods p *ₙ* ". For this reason, to be able to measure the angle in a wide range and with high accuracy, it is necessary to use the optical structure 2 consisting of several lattices. The several lattices can have relatively large common period *p* (the superlattice period), or, otherwise, can have no period at all (the structure is aperiodic and does not replicate itself in space), which makes it possible to obtain non-replicated moire pattern in a wide range of angles, but at the same time with rather fine unique features. The described approach, being one of the embodiments of multiscale structures, makes it possible to provide high measurement accuracy within a wide range.

For simplicity of the example, this patent describes a series of strictly periodic lattices whose periods are proportional to the powers of two. Nevertheless, the required properties can be inherent in series of lattices with other periods, including the random ones, gradient lattices, weakly aperiodic lattices, quasilattices, and many other structures of a short-range order. In this particular example, the large period lattices and respective moire fringes are most useful for a coarse estimation of the angle of rotation of the optical structure 2. At the same time, fine lattices are far more sensible to rotations and can be used to measure the angles more accurately.

When observing the pattern along the normal to its surface, bright fringes are seen for all the lattices, as all the lattices are aligned relative to the origin and the "lines" in the upper lattices do not overlap the "slits" in the lower lattices. Let us denote the value of the n-th fringe intensity as *i ₙ*(*α*), and assume that for the bright fringe *i =* 1, and for the dark fringe i = 0. Within a simplest theoretical model, it is possible to assume that the moire fringe intensity changes between the maximum and minimum values in accordance with a harmonic law. Within this approximation, we will neglect the high-order Fourier harmonics whose magnitude in practice is relatively small indeed. Thus, the theoretical estimation will give us:
[Math. 3] ${i}_{n} \left(α\right) = \frac{1}{2} + \frac{1}{2} cos \left(2π\frac{H tg α}{{p}_{n}}\right) = {cos}^{2} \left(π\frac{H tg α}{{p}_{n}}\right) = {cos}^{2} \left(π\frac{tg α}{{p}_{n}^{a}}\right)$

Thus, in practice, we retrieve *N* of numbers *i ₙ* from the photograph, and the task we have is to reconstruct the angle *α* at which the respective intensities are realized. From the mathematical point of view, we are dealing with an overdetermined system of equations. The system has to be overdetermined in order to be tolerant to inaccuracies and to deliberately avoid possible ambiguities in the solution (several angle values that satisfy the equation).

The most common approach to solving overdetermined systems of equations is the least squares method. As applied to our task, this method consists in finding the angle value at which the following expression will be minimized:
[Math. 4] ${α}_{recovered} = {argmin}_{α ∈ \left[0°,90°\right]} {\sum }_{n} {\left({i}_{n}^{photo}-{i}_{n}\left(α\right)\right)}^{2}$
where i *ₙ*^{photo} is the intensity of the n-th lattice in the photograph.

[Fig.12] shows an image built in a graphics editor and representing the optical structure 2 consisting of a pair of identical lattices having the sublattices with the period *p ₙ* = 5 • 2^{*n*-1} µm, n ∈{ 1,2,3,4,5,6,7} spaced *H =* 200 µm apart. This optical structure 2 is rotated 15 degrees about one of the axes relative to the camera 3. Using the obtained image, we retrieved the respective values of the intensity i *ₙ*^{photo} and built the dependency of the theoretical intensity estimation deviation from the measured magnitudes as a function of the rotation angle:
[Math. 5] ${f}_{RMS} \left(α\right) = {\sum }_{n} {\left({i}_{n}^{photo}-{i}_{n}\left(α\right)\right)}^{2}$

If the data from the photograph had been retrieved with an ideal accuracy, for the angle value to be accurate, the deviation value should have become strictly zero. An unavoidable inaccuracy results in that the curve dip does not reach strict zero, but nevertheless its position can and should be used to determine the rotation angle.

As it can be seen in [Fig.17], the function *f*_{RMS} demonstrates an expected dip at about 15 degrees, and also a whole series of secondary dips for the angles above 50 degrees. The reason is that at the corresponding angle values the set of the resulting parameters i *ₙ*^{photo} is identical or very close to the set that gives the rotation angle of 15 degrees. Since the angles at which the side peaks are observed significantly deviate (are strongly detuned) from the correct value, we can easily select the "correct" peak. For example, based on the general image on the camera, which is the image used to record the moire pattern, it is easy to distinguish a rotation angle of about 15 degrees from a rotation angle above 50 degrees using standard techniques.

At the same time, a more detailed analysis of the dip line position [Fig.18] gives the angle estimation of 14.98 degrees. This value falls within the expected inaccuracy range which is equal to half a period of the most frequent moire
[Math. 6] ${p}_{1}^{α} / 2 = 0.72 °$

Thus, it has been practically proved that using the disclosed method and embodiments of a device in the form of a contact lens makes it possible to significantly simplify implementation of the user's eye tracking and significantly enhance the eye tracking accuracy.

## Claims

1. A user's eyes tracking method, wherein a camera (3) is installed that is configured to monitor the user's eyes, at least on the user's one eye a contact lens (1) is placed that is equipped with an optical structure (2) forming a stereoscopic image, and the user's eyes position is determined from the linear dimension of this image recorded by the camera (3) and from the angular position of this image relative to the optical axis of said camera (3).

2. The method according to claim 1, **characterized in that** said optical structure is implemented as an autostereogram and a decoding element that are separated by a layer of transparent material.

3. The method according to claim 1, **characterized in that** said optical structure is implemented as a hologram.

4. The method according to claim 1, **characterized in that** said optical structure is implemented in the form of at least two segments located along the contact lens perimeter, the angular position of the formed stereoscopic image is determined based on each of said segments, and the obtained data is used to determine the contact lens bending radius.

5. A contact lens (1) containing a central region that in operative position is located opposite the user's pupil (4) and a peripheral region located outside the user's field of view, at least in part made of a transparent material, and equipped with an optical structure (2), **characterized in that** said optical structure (2) is implemented as an autostereogram and a decoding slit raster (5) that is formed on the outer surface of the contact lens (1) peripheral region and configured to form stereoscopic image encoded in said autostereogram, wherein the autostereogram is formed by reflecting said slit raster (5) in a mirror coating (8) applied on the inner surface of the contact lens (1) peripheral region, or said autostereogram is formed by a shadow cast by said slit raster (5) on a diffusely reflecting coating (8) applied on the inner surface of the contact lens (1) peripheral region.

6. A contact lens (1) containing a central region that in operative position is located opposite the user's pupil (4) and a peripheral region located outside the user's field of view, at least in part made of a transparent material, and equipped with an optical structure (2), **characterized in that** said optical structure (2) is implemented as an autostereogram and a decoding raster (5) configured to form stereoscopic image encoded in said autostereogram, which are separated by a layer of transparent material, wherein said autostereogram and/or decoding raster (5) are formed as a real image comprised of replicated elements with the period p and at least two different characteristic linear dimensions, at least one of which is *d ₁* <*1*/*2·p*.

7. The contact lens according to claim 6, **characterized in that** said replicated elements are configured to form stereoscopic image adapted to be used as a goniometric scale.

8. The contact lens according to claim 6, **characterized in that** said peripheral region of the contact lens extends beyond the user's eye iris.

9. A contact lens (1) adapted to implement the method of claim 1 and containing a central region that in operative position is located opposite the user's pupil (4) and a peripheral region located outside the user's field of view, at least in part made of a transparent material, and equipped with an optical structure (2), **characterized in that** said optical structure (2) is implemented as a hologram recorded for a given light wavelength and configured to form a stereoscopic image when the contact lens (1) is illuminated by a beam with said light wavelength.

## Patentansprüche

1. Verfahren zur Verfolgung des Auges eines Benutzers, wobei eine Kamera (3) installiert ist, die so ausgebildet ist, dass sie die Augen des Benutzers überwacht, wobei zumindest auf einem Auge des Benutzers eine Kontaktlinse (1) platziert wird, die mit einer ein stereoskopisches Bild erzeugenden optischen Struktur (2) ausgestattet ist, und die Position der Augen des Benutzers aus der linearen Abmessung dieses von der Kamera (3) aufgezeichneten Bildes und aus der Winkelposition dieses Bildes relativ zu der optischen Achse der Kamera (3) bestimmt wird.

2. Verfahren nach Anspruch 1, **dadurch gekennzeichnet, dass** die optische Struktur als ein Autostereogramm und ein Dekodierelement implementiert ist, die durch eine Schicht aus transparentem Material voneinander getrennt sind.

3. Verfahren nach Anspruch 1, **dadurch gekennzeichnet, dass** die optische Struktur als ein Hologramm implementiert ist.

4. Verfahren nach Anspruch 1, **dadurch gekennzeichnet, dass** die optische Struktur in der Form von zumindest zwei Segmenten implementiert ist, die entlang des Umfangs der Kontaktlinse angeordnet sind, die Winkelposition des erzeugten stereoskopischen Bildes auf der Grundlage jedes der Segmente bestimmt wird und die erhaltenen Daten zur Bestimmung des Krümmungsradius der Kontaktlinse verwendet werden.

5. Kontaktlinse (1), aufweisend einen zentralen Bereich, der in einer Betriebsposition gegenüber der Pupille (4) des Benutzers angeordnet ist, und einen peripheren Bereich, der außerhalb des Sichtfelds des Benutzers angeordnet ist, zumindest teilweise aus einem transparenten Material bestehend und mit einer optischen Struktur (2) ausgestattet, **dadurch gekennzeichnet, dass** die optische Struktur (2) als ein Autostereogramm und ein Dekodierspaltraster (5) implementiert ist, das auf der Außenfläche des peripheren Bereichs der Kontaktlinse (1) ausgebildet und so ausgebildet ist, dass es das in dem Autostereogramm kodierte stereoskopische Bild erzeugt,
wobei das Autostereogramm durch Reflexion des Schlitzrasters (5) in einer auf der Innenfläche des peripheren Bereichs der Kontaktlinse (1) aufgebrachten Spiegelbeschichtung (8) ausgebildet wird oder das Autostereogramm durch einen Schatten ausgebildet wird, den das Schlitzraster (5) auf eine auf der Innenfläche des peripheren Bereichs der Kontaktlinse (1) aufgebrachte diffus reflektierende Beschichtung (8) wirft.

6. Kontaktlinse (1), aufweisend einen zentralen Bereich, der in einer Betriebsposition gegenüber der Pupille (4) des Benutzers angeordnet ist, und einen peripheren Bereich, der außerhalb des Sichtfelds des Benutzers angeordnet ist, zumindest teilweise aus einem transparenten Material bestehend und mit einer optischen Struktur (2) ausgestattet,
**dadurch gekennzeichnet, dass** die optische Struktur (2) als ein Autostereogramm und ein Dekodierraster (5) implementiert ist, das so ausgebildet ist, dass es das in dem Autostereogramm kodierte stereoskopische Bild erzeugt, welches durch eine Schicht aus transparentem Material voneinander getrennt ist, wobei das Autostereogramm und/oder
das Dekodierraster (5) als ein reales Bild ausgebildet sind, das aus wiederholten Elementen mit der Periode p und zumindest zwei unterschiedlichen charakteristischen linearen Abmessungen besteht, von denen zumindest eine d₁ < 1/2·p ist.

7. Kontaktlinse nach Anspruch 6, **dadurch gekennzeichnet, dass** die wiederholten Elemente so ausgebildet sind, dass sie ein stereoskopisches Bild erzeugen, das angepasst ist, um als eine goniometrische Skala verwendet zu werden.

8. Kontaktlinse nach Anspruch 6, **dadurch gekennzeichnet, dass** sich der periphere Bereich der Kontaktlinse über die Iris des Benutzers hinaus erstreckt.

9. Kontaktlinse (1), die zur Implementierung des Verfahrens nach Anspruch 1 angepasst ist und einen zentralen Bereich aufweist, der in einer Betriebsposition gegenüber der Pupille (4) des Benutzers angeordnet ist, sowie einen außerhalb des Sichtfelds des Benutzers angeordneten peripheren Bereich, zumindest teilweise aus einem transparenten Material bestehend und mit einer optischen Struktur (2) ausgestattet,
**dadurch gekennzeichnet, dass** die optische Struktur (2) als ein für eine bestimmte Lichtwellenlänge aufgezeichnetes Hologramm implementiert und so ausgebildet ist, dass sie ein stereoskopisches Bild erzeugt, wenn die Kontaktlinse (1) von einem Strahl mit dieser Lichtwellenlänge beleuchtet wird.

## Revendications

1. Procédé de suivi des yeux d'un utilisateur, dans lequel une caméra (3) est installée qui est configurée pour surveiller les yeux de l'utilisateur, sur au moins un œil de l'utilisateur une lentille de contact (1) est placée qui est équipée d'une structure optique (2) formant une image stéréoscopique, et la position des yeux de l'utilisateur est déterminée à partir de la dimension linéaire de cette image enregistrée par la caméra (3) et à partir de la position angulaire de cette image par rapport à l'axe optique de ladite caméra (3).

2. Procédé selon la revendication 1, **caractérisé en ce que** ladite structure optique est mise en œuvre sous la forme d'un autostéréogramme et d'un élément de décodage qui sont séparés par une couche de matériau transparent.

3. Procédé selon la revendication 1, **caractérisé en ce que** ladite structure optique est mise en œuvre en tant qu'hologramme.

4. Procédé selon la revendication 1, **caractérisé en ce que** ladite structure optique est mise en œuvre sous la forme d'au moins deux segments situés le long du périmètre de la lentille de contact, la position angulaire de l'image stéréoscopique formée est déterminée sur la base de chacun desdits segments, et les données obtenues sont utilisées pour déterminer le rayon de courbure de la lentille de contact.

5. Lentille de contact (1) contenant une région centrale qui, en position opératoire, est située en regard de la pupille (4) de l'utilisateur et une région périphérique située en dehors du champ de vision de l'utilisateur, réalisée au moins en partie en un matériau transparent, et équipée d'une structure optique (2), **caractérisée en ce que** ladite structure optique (2) est mise en œuvre sous la forme d'un autostéréogramme et d'une trame (5) de fentes de décodage qui est formée sur la surface externe de la région périphérique de la lentille de contact (1) et configurée pour former une image stéréoscopique codée dans ledit autostéréogramme,
dans laquelle l'autostéréogramme est formé en réfléchissant ladite trame (5) de fentes dans un revêtement (8) miroir appliqué sur la surface interne de la région périphérique de la lentille de contact (1), ou ledit autostéréogramme est formé par une ombre projetée par ladite trame (5) de fentes sur un revêtement (8) réfléchissant de manière diffuse appliqué sur la surface interne de la région périphérique de la lentille de contact (1).

6. Lentille de contact (1) contenant une région centrale qui, en position opératoire, est située en regard de la pupille (4) de l'utilisateur et une région périphérique située en dehors du champ de vision de l'utilisateur, réalisée au moins en partie en un matériau transparent, et équipée d'une structure optique (2),
**caractérisée en ce que** ladite structure optique (2) est mise en œuvre sous la forme d'un autostéréogramme et d'une trame (5) de décodage configurée pour former une image stéréoscopique codée dans ledit autostéréogramme, lesquels sont séparés par une couche de matériau transparent, dans laquelle ledit autostéréogramme et/ou
ladite trame (5) de décodage sont formés en tant qu'image réelle comprenant des éléments répliqués avec la période p et au moins deux dimensions linéaires caractéristiques différentes, dont au moins une est d₁ < 1/2·p.

7. Lentille de contact selon la revendication 6, **caractérisée en ce que** lesdits éléments répliqués sont configurés pour former une image stéréoscopique apte à être utilisée en tant qu'échelle goniométrique.

8. Lentille de contact selon la revendication 6, **caractérisée en ce que** ladite région périphérique de la lentille de contact s'étend au-delà de l'iris de l'œil de l'utilisateur.

9. Lentille de contact (1) apte à mettre en œuvre le procédé de la revendication 1 et contenant une région centrale qui, en position opératoire, est située en regard de la pupille (4) de l'utilisateur et une région périphérique située en dehors du champ de vision de l'utilisateur, réalisée au moins en partie en un matériau transparent, et équipée d'une structure optique (2),
**caractérisée en ce que** ladite structure optique (2) est mise en œuvre sous la forme d'un hologramme enregistré pour une longueur d'onde lumineuse donnée et configurée pour former une image stéréoscopique lorsque la lentille de contact (1) est éclairée par un faisceau présentant ladite longueur d'onde lumineuse.
